# EUROPEAN PATENT APPLICATION

(11) **EP 0 882 446 A1**
(43) Date of publication of application: **09.12.1998**
(21) Application number: 97303693.2
(22) Date of filing: 02.06.1997
(51) Int. Cl.: A61K 7/48, A61K 7/50

(54) **Antimicrobial skin cleansing compositions**

(71) Applicant: GOJO INDUSTRIES,INC., Cuyahoga Falls OH 44223 (US)
(72) Inventor: Fendler, Eleanor J., Hudson, Ohio 44236 (US); Williams, Ronald A., Stow, Ohio 44224 (US); Comes, Demetrius A., Akron, Ohio 44311 (US); Dolan, Michael J, Akron,Ohio 44333 (US)
(74) Representative: Rackham, Anthony Charles

(57) **Abstract**

A cleansing composition includes a substituted phenol such as para-chloro-meta-xylenol or triclosan, and at least one primary surfactant selected from the group consisting of amine oxides, phospholipids, partially neutralized carboxylic acids and diacids, betaines, ethoxylated methyglucosides, and mixtures thereof. The composition is devoid of conventional anionic and nonionic surfactants. Other additives such as viscosifiers or thickeners, emollients, fragrances, perfumes, coloring agents, and the like may also be added. The cleansing composition has been found to exhibit improved antibacterial properties while remaining mild on the skin.

## Description

### TECHNICAL FIELD

This invention relates generally to skin cleansing compositions. More particularly, the present invention relates to antimicrobial skin cleansing compositions containing substituted phenols such as triclosan or para-chloro-meta-xylenol (PCMX) as the active antimicrobial compound in the compositions. Specifically, the present invention relates to an antimicrobial skin cleansing composition containing substituted phenol, *e.g.*, triclosan, PCMX, etc., and at least one primary surfactant selected from the group consisting of amine oxides, phospholipids, partially neutralized carboxylic acids and diacids, betaines, ethoxylated methylglucosides, and mixtures thereof. Notably, the antimicrobial cleansing compositions of the present invention are devoid of conventional surfactants such as most nonionic and anionic surfactants (*e.g.*, fatty acid soaps, natural soap, etc.) and other substances commonly used therein (*e.g.*, polyethylene glycol stearate, alcohol, etc.) which have been found to inhibit the antimicrobial activity of the substituted phenol. Furthermore, the antimicrobial cleansing compositions do not necessarily require ethylenediaminetetraacetic acid (EDTA) or non-aqueous carriers or diluents.

### BACKGROUND OF THE INVENTION

Antibacterial cleansing compositions are typically used to cleanse the skin of the user and to destroy bacteria ad ay other microorganisms which might be present on the user's skin. These compositions are widely used in the health care industry by hospital staff and other health care personnel as hand washing cleansers to prevent nosocomial infections. They are particularly suitable for use by hospital personnel such as surgeons, nurses and other health care professionals who might be subject to contact with bacteria and the like. They are also suitable for use by personnel in the food service and meat processing industries and, generally are used for antimicrobial cleansing of the hands and arms by the public.

There are several active antimicrobial agents currently available for use in skin cleansing compositions. For example, may antimicrobial skin cleansing compositions contain a bisbiguanide bacterial substance such as chlorhexidine digluconate (CHG). Other skin cleansing compositions use phenolic compounds. The antimicrobial activity of these substances, however, are often dependent upon the type(s) of surfactants or other ingredients employed therewith, and therefore, the use of the same ingredients with these antimicrobial substances will not necessary derive the same result. For instance, where CHG is employed as the active antimicrobial agent, the cleansing compositions typically includes alkyl polyglucosides and nonionic alcohol ethoxylates as the primary surfactants. However, in compositions containing substituted phenols such as triclosan or PCMX as the active antimicrobial agent, most of the nonionic alcohol ethoxylates inhibit, rather than enhance, the antibacterial activity of the active agent.

Heretofore, antimicrobial skin cleansing compositions containing substituted phenols as the active antimicrobial agent have also included anionic detergents, soaps, surfactants, and the like, as well as other compounds (nonionic ethoxylated surfactants, polyethylene glycol, isopropyl alcohol and other lower alkanols, etc.) which are known to substantially reduce the antibacterial activity of the phenolic compounds. For example, U.S. Pat. No. 3,824,190 and its subsequent Reissue Pat. No. Re. 28,778 to Winicov et al. both disclose the use of substituted phenolic compounds and anionic surface active detergents or soaps in an alcohol and water-based carrier. Additionally, chelating agents such as ethylenediaminetetraacetic acid (hereinafter, EDTA) were also added.

Other patents also disclose the use of phenolic-based compounds such as, for example, 2,4,4'-trichloro-2'-hydroxydiphenyl ether (triclosan) ad 4-chloro-3,5-dimethylphenol (PCMX). However, in each of these patents, at least one of the additives tends to inhibit the antimicrobial activity of the preferred antimicrobial agent. In any instance where this is not the case, then the antimicrobial agent is typically not the active ingredient in the composition. That is, it is well known to use many of these antimicrobial agents at preservative levels far below that required for effective antimicrobial action. In these instances, other antimicrobial active ingredients are included which effectively neutralize the organisms, and the preservatives serve only to increase the shelf life of the product.

Substituted phenols and particularly, para-chloro-meta-xylenol (PCMX) have been used for decades in Europe and more recently in the United States as an active ingredient in antiseptics and hand washing agents for health care personnel. PCMX, also listed in the Merck Index as 4-chloro-3,5-dimethyl phenol, is a substituted phenol having the structure 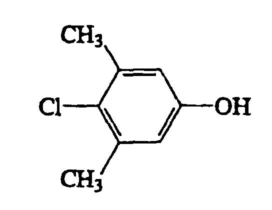 and is reported to have 60 times the antimicrobial activity of phenolic against a wide spectrum of bacteria. Moreover, PCMX has been reported to yield high initial reductions of Gram-positive and Gram-negative bacteria as well as fungi, and also to provide good residual activity for several hours between hand washings.

Similarly, triclosan, or 5-chloro-2-(2,4 dichlorophenoxy)phenol as it is also noted the Merck Index, has long been used in the United States as an active ingredient in certain medicated skin cremes and preparations. This chlorophenol-substituted phenol has the structure

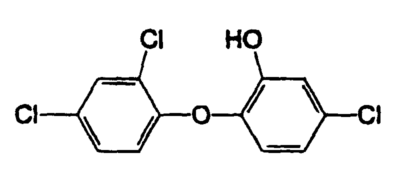

Thus, while the use of a substituted phenols such as triclosan and PCMX as an active antimicrobial agent in a skin cleansing composition would appear to be well known, it is believed unknown to use these antimicrobial agents in conjunction with certain additives and devoid of other additives such that the antimicrobial activity of the agent is not reduced substantially and, in some cases, may even be enhanced. Typically, where triclosan or PCMX is used in a cleansing composition, it is used in concentrations of from about 0.1 to 4 perent by weight. However, the majority of formulas contain 1 percent or less by weight.

In other words, as discussed briefly hereinabove, like that of chlorohexidene digluconate (CHG), the efficacy of triclosan, PCMX and other substituted phenols against microorganisms is highly dependent upon the carrier and other chemical constituents of the antimicrobial cleanser. For instance, it has been reported that the antimicrobial activity of PCMX is potentiated against *Pseudomonas aeruginosa* by the addition of the chelating agent ethylenediaminetetraacetic acid (EDTA) and/or the sequestering agent sodium hexametaphosphate, presumably due to bonding of the agents to metal ions in the cell wall. On the other hand, the antimicrobial activity of PCMX has been reported to be reduced in the presence of organic material, and may be inhibited by moderate concentrations of anionic surfactants. Antimicrobial activity is also lost as a consequence of reversible interactions between PCMX and noninonic surfactants, polyethylene glycol, and polyethylene glycol stearate. Similarly, reduction in the antibacterial efficacy of triclosan and PCMX has also been found to be the direct result of its interaction with a variety of macromolecules, namely, methyl cellulose, polyethylene glycol 6000, and polysorbate 80. The importance of these chemical and biochemical interactions cannot be over-stated because they determine the biocidal effectiveness of triclosan and PCMX in hand washing formulations and other antimicrobial products.

Accordingly, the need exists for an antibacterial composition containing a substituted phenol as the active antimicrobial ingredient therein, which is suitably effective for killing bacteria and other microorganisms, but which does not include conventional anionic surfactants or other chemical ingredients, including but not limited to certain nonionic surfactants, known to be detrimental to the antimicrobial activity of the substituted phenol. That is, the desired skin cleansing composition should include surfactants and carriers which do not significantly effect (i.e., reduce) the antimicrobial activity of the substituted phenol.

### SUMMARY OF INVENTION

It is therefore an object of the present invention to provide a skin cleansing composition which effectively kills bacteria and other microorganisms.

It is another object of the present invention to provide a skin cleansing composition, as above, which is devoid of conventional anionic surfactants.

It is still another object of the present invention to provide a skin cleansing composition, as above, which includes a substituted phenol such as triclosan or PCMX and at least one primary surfactant.

At least one or more of the foregoing objects, together with the advantages thereof over the known art relating to antimicrobial cleansing compositions, which shall become apparent from the specification which follows, are accomplished by the invention as hereinafter described and claimed.

In general, the present invention provides an antimicrobial cleansing composition including a substituted phenol and at least one primary surfactant selected from the group consisting of amine oxides, phospholipids, partially neutralized carboxylic acids and diacids, betaines, ethoxylated methylglucosides, and mixtures thereof. The composition may also preferably include viscosifiers, chelating and sequestering agents, emollients, humectants and other ingredients which do not materially affect the antimicrobial efficacy of the composition.

### PREFERRED EMBODIMENT FOR CARRYING OUT THE INVENTION

The present invention is directed toward an antibacterial skin cleansing composition which effectively kills bacteria and other microorganisms, but is otherwise mild on the skin such that it does not cause skin irritation or dryness. That is, the antimicrobial cleansing compositions of the present invention have been found to reduce significantly the number of colony forming units (cfu's) of bacteria such as *Staphylococcus aureus* and the like and to do so without the use of any conventional anionic surfactants or other ingredients known to reduce the efficacy of the active antimicrobial agent employed therein.

The essential ingredients for such an antimicrobial cleansing composition generally includes a substituted phenol and at least one primary surfactant. These ingredients, along with other additives disclosed hereinbelow, provide compositions with excellent disinfecting properties as well as excellent cleansing properties, while remaining mild with respect to skin irritation or dryness.

As noted hereinabove and as is generally known in the art, the antibacterial activity for the compositions is generally provided by the substituted phenol found therein. Preferably, these substituted phenols are used in amounts ranging from about 0.1 to about 4 percent by weight of the total composition, with about 0.3 to 1 percent by weight of the total composition being most preferred. It will be appreciated, however, that more than 4 percent by weight of the antimicrobial agent may be employed without departing from the spirit of the invention, the upper limit being based solely upon the amount of substituted phenol which is considered to be non-toxic and non-irritating to the skin. The lower limit may include less than 0.1 percent to the extent that such amounts would still be antimicrobially effective. However, lesser amounts do not include amounts where the substituted phenol is no longer consider the antimicrobial agent. That is, preservative levels of these substituted phenols are insufficient to be antimicrobially effective.

Any substituted phenol which is preferably soluble in water or other non-alkanol solvent (triethylene glycol, propylene glycol, hexylene glycol etc.) may be used in the compositions of the present invention. Preferred substituted phenols include alkyl substituted, halogen substituted, phenol substituted, halophenol substituted, alkyl halo substituted, benzyl alkyl substituted, phenylethyl alkyl substituted and benzyl halo substituted phenols, and alkyl substituted, halogen substituted and alkyl halo substituted bisphenols, bis(hydroxyphenyl) alkanes, amino substituted phenols, resorcinol derivatives, trihydric phenol derivatives, naphthol derivatives and carboxylic acid substituted phenols (*e.g.*, salicylic acid and esters). Most preferred of the substituted phenols is a chlorophenol-substituted phenol, namely triclosan.

Also preferred is a chloro-substituted phenol, namely para-chloro-meta-xylenol (PCMX). As noted hereinabove, PCMX is reported to have 60 times the antimicrobial activity of phenol against a wide spectrum of bacteria. It has been reported to yield high initial reductions of Gram-positive and Gram-negative bacteria as well as fungi, and also to provide good residual activity for several hours between hand washings. It has also considered to be non-toxic and non-irritating to human skin at levels below about 3 percent by weight.

In the samples presented hereinbelow, Triclosan and PCMX are the only substituted phenols employed therein. However, the present invention should not necessarily be limited to these particular substituted phenols as other such substituted phenols as noted hereinabove may also be suitable for the purposes of the present invention.

As noted hereinabove, at least one primary surfactant is also incorporated into the composition of the present invention. As used in this specification, the term "primary surfactant" means any surfactant which acts upon or in conjunction with the substituted phenol to enhance further, or at the least, to not reduce significantly, the antimicrobial activity of the substituted phenol. Accordingly, insofar as conventional anionic surfactants have been found to reduce substantially the antimicrobial activity of substituted phenols, they are not primary surfactants for purposes of this invention. One of ordinary skill in the art would readily recognize whether a particular surfactant is a primary surfactant or not without undue experimentation. Examples of conventional anionic surfactants generally include, but are not necessarily limited to, fatty acid soaps as well as sulfates, carboxylates, sulfonates, sulfosuccinates, phosphonates, phosphates, sarcosinates, and isethionates of hydrophobic moities. Moreover, as noted hereinabove, many nonionic surfactants also inhibit the antibacterial activity of substituted phenols. Accordingly, only the particular nonionic surfactants noted hereinbelow may be employed.

In order to overcome the problem of inhibition of the active antimicrobial ingredient, the composition of the present invention preferably includes primary surfactants selected from the group consisting of amine oxides, phospholipids, partially neutralized carboxylic acids and diacids, betaines, ethoxylated methylglucosides, and mixtures thereof. The amount of primary surfactant(s) to be added to the composition of the present invention is somewhat dependent upon the number of primary surfactants added. However, the amount of all of the primary surfactants together generally and preferably will not comprise more than about 20 percent by weight of the composition.

With respect to the amine oxides, any amine oxide may be employed, but preferably, an alkyl amine oxide is added to the composition in an amount ranging from 0 (absent) to about 10 percent by eight with about 5 to 7 percent by weight being most preferred. When the amine oxide is the sole primary surfactant employed in the composition, it is preferably added in amounts ranging from about 1 to about 10 percent by weight. Preferably, the amine oxide has a alkyl chain length of from about C₈ to about C₁₆, with chain lengths of from C₁₂ to C₁₄ being most preferred. Heretofore, the amine oxides were typically employed in cleansing formulations as secondary surfactants in conjunction with conventional anionic surfactants as the major or primary surfactant for cleaning and foam-enhancing purposes. While they are used for the same purposes in the present invention, it will be appreciated that they are no longer secondary surfactants. That is, the amine oxides of the present invention are generally the major surfactant in the composition. No conventional anionic surfactants are used.

The amine oxides used in the present invention are considered to be amphoteric surfactants. Examples of these amine oxides found to be suitable for the present invention include those available from McIntyre Chemical Co., Ltd. of Chicago, Illinois, under the tradenames Mackamine LO (a C₁₂ alkyl amine oxide also known as lauramine oxide) and Mackamine CO (a C₁₂-C₁₄ alkyl amine oxide also known as cocamine oxide), as well as those available from Lonza, Inc., of Fair Lawn, New Jersey, under the tradenames Barlox 14 (a C₁₄ alkyl amine oxide) and Barlox 12 (a C₁₂ alkyl amine oxide). Furthermore, amidopropyl amine oxides such as those available from Henkel Corp. of Hoboken, New Jersey, under the tradenames Standamox LAO and Mackamine CAO may be employed.

In addition, alkylamides such as cocamide may be used in conjunction with the amine oxides or other primary surfactants to boost foaming and add to the viscosity of the composition. Preferred viscosity modifiers and foam stabilizers are the alkylamides which are also available from Henkel Corp. under the tradenames Standamide CD, Standamide SD, and Cocamide DEA.

These compounds are seen as being particularly effective in use with substituted phenols. That is, they do not significantly reduce the antibacterial activity of the substituted phenol and particularly have been found to be extremely effective with PCMX in killing *Staphylococcus aureus* and other microorganisms as detailed hereinbelow.

Other zwitterionic/amphoteric surfactants such as phospholipids and betaines may also be used in the compositions of the present invention. These surfactants are also considered to be primary surfactants as defined hereinabove and may be used in place of or in conjunction with the amine oxides noted hereinabove. When used, the phospholipids and betaines may be employed in amounts up to about 10 percent by weight.

One particular class of phospholipids suitable for use in the cleansing compositions of the present invention is alkyl phosphatidyl PG-dimonium chloride. The preferred alkyl phosphatidyl PG-dimonium chlorides are cocamidopropyl phosphatidyl PG-dimonium chloride, available from MONA Industries, Inc. of Paterson, New Jersey, under the tradename Phospholipid PTC; stearamidopropyl phosphatidyl PG-dimonium chloride, available from MONA under the tradename Phospholipid PTS; linoleamidopropyl phosphatidyl PG-dimonium chloride, available from MONA under the tradename Phospholipid EFA; and stearamidopropyl phosphatidyl PG-dimonium chlorid/cetyl alcohol, available from MONA under the tradename Phospholipid SV. Preferably, such compounds are incorporated into the composition in an amount ranging from 0 (absent) to about 10 percent by weight of the total composition.

Additionally, betaines may also be used as the primary surfactant either alone or in conjunction with other primary surfactants identified herein. Preferably, up to 10 percent by weight of the total composition of an alkylammonio carboxylate having from 8 to 18 carbon atoms may be incorporated into the composition. One such alkylammonio carboxylate is cocobetaine, and is available from McIntyre Chemical Co. of Chicago, Illinois, under the tradename Mackam CB-35.

Still further, mixtures of partially neutralized carboxylic acids and diacids, *e.g.*, sodium caproyl lactylate, may be employed as the primary surfactant. Preferably, such a compound is added to the cleansing composition in amounts ranging from 0 (absent) to about 5 percent by weight. At this point, it is noted that these partially neutralized carboxylic acids and diacids contain some anionic surfactants. However, such mixtures of these acid salts have considerable protonated acid character and therefore, are expressly excluded from the term "conventional anionic surfactants".

Notwithstanding the above surfactants, at least one type of nonionic surfactant, ethoxylated nonionic methylglucosides, may be used in the present invention. However, not all nonionic ethoxylates are suitable for this invention. In fact, the only ethoxylates known to date to maintain the desired anti-microbial activity of the substituted phenols are ethoxylated (10 moles) methylglucosides and ethoxylated (20 moles) methylglucosides. These ethoxylates may also be added in amounts ranging from 0 (absent) to about 10 percent by weight.

The composition may also include other additives such as thickeners, emollients, chelating and sequestering agents, fragrances, coloring agents, opacifying agents, pearlizing agents, vitamins and the like. For example, the composition may include a polymer viscosifier or thickener such as hydroxyethyl cellulose to make the composition more aesthetically pleasing. Examples of other suitable polymer viscosifiers include, but are not necessarily limited to, hydroxypropyl cellulose, methyl cellulose, and carboxymethyl cellulose.

A glycol may be added in order to quicken the rate of dissolution of the substituted phenol and as an emollient and/or humectant. Preferred glycols include propylene glycol, triethylene glycol, and hexylene glycol, with propylene glycol being most preferred. These glycols may be added in amounts raging from 0 (absent) to about 10 percent by weight of the total composition, with about 3 percent being most preferred. Preferably, the PCMX or the substituted phenol is dissolved in at least a portion of the glycol prior to adding other ingredients to the solution. A 25 percent/75 percent ratio of PCMX to glycol is most preferred.

Still further, up to about 1 percent by weight of a chelating agent and/or sequestering agent may be added to soften the water-based composition. However, it will be appreciated that, like the other additives, these agents have no demonstrable effect on the antimicrobial activity of the substituted phenol compound employed. One example of a suitable chelating agent for the present invention is ethylenediaminetetra-acetic acid (EDTA). Most preferably, disodium EDTA is employed.

The composition of the present invention may further comprise minor but effective amounts of other conventional additives such a fragrances, color additives, opacifying agents, pearlizing agents, vitamins, etc. An example of a particular pearlizing agent includes, but is not necessarily limited to, ethylene glycol distearate. Generally, these additives are used in amounts which do not affect the essential nature of the composition with respect to its antimicrobial properties.

For optimal antibacterial efficacy, the pH of the composition should be between 4 and 8, and preferably between 5.5 and 6.5. To adjust the pH of the composition, any acid compatible with the components of the composition can be used. Preferred acids include lactic acid, citric acid, acetic acid, glycolic acid, and gluconic acid, with the first two acids being most preferred. Typically, less than 1 percent by weight of these acids are used to achieve the proper pH balance.

The balance of the composition is typically water so as to provide 100 percent by weight of the composition. Preferably, no alcohol-based solvent in used; although if alcohol is used, it would not materially affect the nature of the composition.

All percents by weight indicated herein are based upon the percent active composition. Thus, for example, where 5 percent by weight lauramine oxide is employed and the lauramine oxide is obtained from the manufacturer or has been diluted into solution so as to comprise a 30 percent active solution, 16.7 percent of the solution will have to be used in order to obtain the 5 percent by weight recommended.

The antimicrobial cleansing compositions of the present invention are generally prepared by dissolving substituted phenol, namely triclosan or PCMX, in glycol as noted hereinabove, and adding this solution to one or more of the primary surfactant discussed hereinabove, in water. More particularly, the primary surfactant or surfactants, and other ingredients (fragrance, chelating agent, pearlizing agent, etc.) are added to the solution with stirring. The pH of the composition is adjusted with lactic acid or similar acid. The thickener/viscosifier is then preferably added and the solution is mixed until it is completely homogeneous. The pH is checked and adjusted again if necessary. This process may be employed with or without the application of heat to enhance hydration of the viscosifier, if required.

In order to demonstrate practice of the present invention, several antimicrobial cleansing compositions containing PCMX were prepared according to the concepts of the present invention as presented hereinabove and were tested to determine their effectiveness against a particular strain of several different bacteria. The challenged bacteria included at least on strain of *Staphylococcus auerus*. The particular strain is on deposit with and is available to the scientific public from the American Type Culture Collection (ATCC), Rockville, Maryland, under Accession No. 33591. The compositions of the present invention were also studied with respect to other strains of bacteria also available to the scientific public from the ATCC under various Accession numbers. For instance, tests against at least one strain of *Serratia marcescens*, available to the scientific public from the ATCC under the Accession No. 14756, at least one strain of *Escherichia coli*, available to the scientific public from the ATCC under the Accession No. 8739, and at least one strain of *Pseudomonas aeruginosa*, available to the scientific public from the ATCC under Accession No. 15442, were also conducted.

Specifically, two prototype PCMX compositions were prepared from 0.3 and 0.5 percent by weight PCMX, respectively, in a 3 percent propylene glycol-USP solvent solution, along with 5 percent by weight of a primary surfactant, namely lauramine oxide (30% active), 0.7 percent by weight of a thickener, namely hydroxyethyl cellulose, and the balance, water. The pH of the compositions were determined and adjusted to 7.0.

The *in vitro* antimicrobial efficacy of these two PCMX compositions were then evaluated and compared to several commercially available PCMX antimicrobial skin cleansers. More particularly, four professional antimicrobial lotion soaps were obtained from local medical distributors and tested against the two antimicrobial soap formulations of the present invention described hereinabove. While the exact compositional amounts of the commercially available antimicrobial lotion soaps may be unknown or proprietary to the manufacturer, the amount (percent by weight) of PCMX in each of the soaps is known and disclosed hereinbelow in Table I. Moreover, upon examination of the ingredients for the commercial antimicrobial products, it was observed that they all contained at least one of ingredient believed to inhibit the antimicrobial activity of PCMX. In contrast, the PCMX formulations based upon the present invention employ only primary surfactants, namely lauramine oxide, which is believed not to exhibit any inhibition to PCMX.

A thirty-second efficacy screen was then conducted using the process detailed hereinbelow. Specifically, approximately forty eight hours prior to performing the efficacy study, each vial of bacteria was rehydrated into a bottle of tryptic soy broth (TBS), and incubated at about 35°C for between 20 and 28 hours (about 24 hours). Each TSA plate was streaked for isolation and incubated at the same temperature for about 24 hours. Immediately prior to performing the efficacy protocol, a bacterial suspension was prepared to a concentration of approximately 1 x 10⁹ cfu's/ml by swabbing the plate culture and suspending the bacteria into a sterile test tube containing 10 ml sterile physiological saline.

Ten milliliter samples of the test solutions were then placed into sterile 30 ml beakers containing a magnetic sir bar. The beaker with the test sample was placed onto a magnetic stirrer and approximately 10 to 15 seconds prior to inoculating the test sample with the challenge bacteria, the stirrer was turned on to allow good mixing without addition of air into the sample. One hundred microliters (µl) of challenge bacteria were introduced into the sample at time 0 and allowed to mix for approximately 20 seconds. Two hundred-fifty µl of the challenged sample was removed with a 250 µl positive displacement pipettor and dispensed at 30 seconds into 24.74 ml of neutralizing broth (2 percent tryptic soy broth (TSB), 10 percent Polysorbate 80, 4 percent Polysorbate 20, 0.75 percent Lecithin, 0.5 percent Dextrose, and 82.75 percent Distilled Water) and mixed well. Serial dilutions were made by standard methods well known in the art and plated into pour plates with tryptic soy agar (TSA) containing Polysorbate 80 and Lecithin (available from BBL). The plates were incubated at 35°C for 48 hours, except for the plates containing *Serratia marcescens* which were incubated at 26°C for 48 hours rather than 35°C. The plates containing 30-300 colonies were counted. The challenge bacterial suspension was enumerated as were the test solutions using 10 ml of Sterile Physiological Saline as the test solution. Moreover, each antimicrobial soap was tested in triplicate for each challenge bacteria and repeated a total of three times for a total of nine replicates.

Each of the four commercial antimicrobial soaps (Compositions 1-4) and the two prototype Compositions, Nos. 5 and 6, were tested against *Staphylococcus aureus* (ATCC Accession No. 33591), *Escherichia coli* (ATCC Accession No. 8739), *Pseudomonas aeruginosa*(ATCC Accession No. 14756), and *Serratia marcescens*(ATCC Accession No. 14756). Table I reports the antibacterial efficacy of reduction of colony forming units (cfu's) of the challenged bacteria in terms of log reduction for a 30 second exposure screen performed against these bacteria and the amount of PCMX in each formulation. The efficacy results have been calculated on a log scale in order to provide a better understanding of the significant improvement in effectiveness on bacteria certain compositions have a compared to other compositions. In percentages, efficacy is considerably less clear insofar a many of these compositions reduce the number of cfu's by greater than 99 percent (a 2 log reduction).

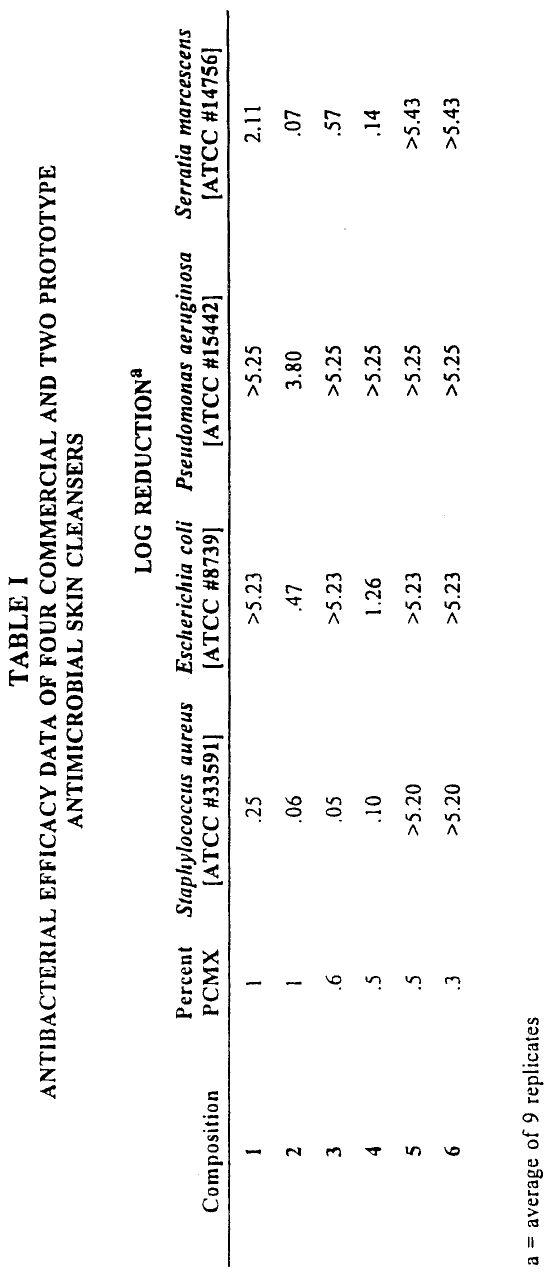

As can be seen in Table I, the antibacterial efficacy of the compositions varies widely with the product and does not parallel the percent concentration of PCMX for the challenge bacteria. These results appear to indicate that the antimicrobial efficacies of antimicrobial skin cleansers are highly dependent upon the formula ingredients. Moreover, it appears that the inhibition of PCMX by formula ingredients can negate the effectiveness of even high concentrations (1 to 3 percent by weight) of PCMX.

Of the commercial products, Composition 1, containing 1 percent by weight PCMX, exhibited the highest antibacterial efficacy, showing greater than 5 log reductions for two of the four challenge bacteria. However, the Compositions 5 and 6 of the present invention containing 0.5 percent by weight and 0.3 percent by weight PCMX, respectively, exhibited greater than 5 log reductions for all four challenge bacteria

Thus, it is believed evident that the *in vitro* antibacterial efficacies of antimicrobial skin cleansers are highly dependent upon the formula ingredients. Moreover, inhibition of PCMX by formula ingredients can negate the effectiveness of PCMX.

It is apparent that a variety of anionic and nonionic surfactants and emollients can significantly inhibit the efficacy of PCMX *in vitro*. The extent of the inhibition is dependent upon both the concentration of the ingredient and on the challenge organism. However, the formulations of the present invention which include a substituted phenol, such as PCMX, and a primary surfactant selected from the group consisting of amine oxides, phospholipids, partially neutralized carboxylic acids and diacids, betaines, ethoxylated methylglucosides, and mixtures thereof produce high (>5 Log) reductions of all four challenge bacteria. While antimicrobial Composition 1 yielded moderate to high log reductions for *Escherichia coli*, *Pseudomonas aeruginosa*, and *Serratia marcescens*. All the other commercial antimicrobial products showed very little if any antibacterial efficacy against the methicillin resistant *Staphylococcus aureus* (Table I). It is apparent from Table I that the *in vitro* antibacterial efficacies of the Compositions 5 and 6 (containing 0.5 percent by weight and 0.3 percent by weight PCMX, respectively) far exceed those of the commercial antimicrobial Compositions 1, 2, 3, or 4 (containing 1.0 percent by weight, 1.0 percent by weight, 0.6 percent by weight and 0.5 percent by weight PCMX, respectively).

The studies, which are presented here, clearly demonstrate the effects of the carrier on the active biocide. Although it is tempting to assume that a product containing a high concentration of PCMX is more effective, the *in vitro* results given clearly demonstrate that this is not true. It is noted that formulation ingredients as well as the concentration of PCMX should be considered in selecting a highly efficacious antimicrobial skin cleanser.

Next, two additional PCMX Compositions (7 and 8) were prepared according to the concepts of the present invention. Each of these compositions are seen as preferred PCMX compositions for the present invention. Of course, it will be appreciated that other additives such a coloring agents, opacifying agents, vitamins or other additives which would not materially affect the composition may be added to these subject compositions without departing from the spirit of the invention. The compositional formulations of each of these compositions are present in Table II hereinbelow.

**TABLE II**

| **ANTIBACTERIAL EFFICACY STUDY PCMX ANTIMICROBIAL FORMULATIONS** | | |
|---|---|---|
| **INGREDIENTS** (tradename) | **COMPOSITION (percent by weight)** | |
| | **7** | **8** |
| Para-Chloro-Meta-Xylenol (PCMX) | .5 | .5 |
| Propylene Glycol USP (PG[USP]) | 3 | 3 |
| Hydroxyethyl cellulose (Natrosol 250 HHR) | .6 | .6 |
| Disodium ethylenediaminetetraacetic acid (N 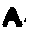 ₂EDTA) | .1 | .1 |
| Cocamine oxide (Mackamine CO) | 4 | 4 |
| Fragrance | .1 | .1 |
| Pearlizing Agent #1 | .5 | -- |
| Pearlizing Agent #2 | -- | .5 |
| Lactic Acid pH = 6.0 | .23 | .23 |
| Water | balance to provide 100 percent by weight | |

A thirty-second efficacy study was also conducted using the process described hereinabove prior to Table I on these two preferred PCMX compositions. Table III reports the antibacterial efficacy in terms of log reduction for a 30-second exposure screen performed against several challenge bacteria including *Staphylococcus aureus* (ATCC 33591) and *Escherichia coli* (ATCC 8739).

**TABLE III**

| **ANTIBACTERIAL EFFICACY SCREEN OF PEARLING AGENTS IN PCMX ANTIMICROBIAL LOTION SOAP** **THIRTY SECOND EXPOSURE KILL STUDY** | | |
|---|---|---|
| **CHALLENGE MICROBE (ATCC Strain)** | **COMPOSITION LOG REDUCTION** | |
| | **7** | **8** |
| *Escherichia coli* (ATCC 8739) | >5.2607 | 5.4255 |
| *Staphylococcus aureus* (ATCC 33591) | 4.4207 | 4.5942 |
| *Staphylococcus aureus* (ATCC 6538) | >5.1319 | -- |
| *Serratia marcescens* (ATCC 14756) | >5.5596 | -- |
| *Salmonella typhimurium* (ATCC 14028) | >5.2355 | -- |
| *Shigella sonnei* (ATCC 11060) | >5.5244 | -- |
| *Proteus mirabilis* (ATCC 7002) | >5.5499 | -- |
| *Klebsiella ozaenae* (ATCC 11296) | >5.4171 | -- |
| *Listeria monocytogenes* (ATCC 7644) | >5.2480 | -- |
| *Enterococcus faecium* (ATCC 19434) | >5.1980 | -- |
| *Pseudomonas aeruginosa* (ATCC 9027) | >5.3570 | -- |
| *Pseudomonas aeruginosa* (ATCC 15442) | >5.5886 | -- |
| *Streptococcus pyogenes* (ATCC 19615) | >4.9445 | -- |
| *Candida albicans* (ATCC 10231) | 3.6532 | -- |

As can be seen, these compositions were highly effective in reducing the colony forming units (cfu's) of the challenged bacteria. Each composition showed at least a 4 log reduction against the bacteria.

Continuing, results of an *in vivo* glove-juice study performed against *Serratia marcescens* (Accession No. 14756) are presented hereinbelow in Table IV. In this test, about five milliliters (5 ml) aliquots of approximately 10⁸/ml *Serratia marcescens* were pipetted into each human subject's cupped hands. The inoculum was then distributed evenly over both hands and part of the forearm by gentle usage. After one minute to ail dry, the subject's hands were washed with the antimicrobial composition of the present invention and rinsed. Excess water was shaken from the hands and powder-free sterile gloves were placed over the hands. A sterile solution was instilled into each glove, and the wrists of the subject were secured. An attendant massaged the hands through the gloves in a standardized manner for about 60 seconds. Aliquots of the glove juice were removed and serially diluted in a tryptic soy broth (TSB). Duplicate spread plates were prepared from each dilution using tryptic soy agar (TSA). The plates were incubated at 20-25°C for approximately 48 hours, and plates having 25-250 colony forming units were counted. This procedure was repeated ten times with a minimum of five minutes between applications and washings. The log reduction from the baseline (0 washes) and log bacterial populations after 0, 1, 5 and 10 washes are provided in Table IV hereinbelow. It can be seen that, based upon the log reduction values obtained for these compositions, the present invention is effective in destroying bacteria and other microorganisms, while at the same time, is mild to the skin.

An additional 15 washes were made to determine the potential for skin irritation. After 25 total washes, skin irritation was found to be negligible.

**TABLE IV**

| **ANTIMICROBIAL LOTION SOAP 0.5% Para-Chloro-Meta-Xylenol** | | | | |
|---|---|---|---|---|
| **Subject** | **Baseline** | **Wash #1** | **Wash #5** | **Wash #10** |
| **3** | 8.80 | 6.52 | 5.23 | 5.47 |
| **6** | 8.71 | 6.29 | 5.88 | 5.01 |
| **8** | 8.02 | 6.97 | 6.08 | 5.43 |
| **Mean Log**_{**10**} **Bacterial Populations** | 8.51 | 6.59 | 5.73 | 5.30 |
| **Mean Log Reduction from Baseline** | -- | 1.92 | 2.78 | 3.21 |

Next, two compositions containing triclosan (Composition Nos. 9 and 10) were prepared according to the concepts of the present invention, Of course, it will be appreciated that other additives such as coloring agents, opacifying agents, vitamins or other additives which would not materially affect the composition may be added to these subject compositions without departing from the spirit of the invention. The compositional formulations of these compositions are present in Table V hereinbelow.

**TABLE V**

| **ANTIBACTERIAL EFFICACY STUDY TRICLOSAN ANTIMICROBIAL FORMULATION** | | |
|---|---|---|
| **INGREDIENTS** (tradename) | **COMPOSITION (percent by weight)** | |
| | **9** | **10** |
| Triclosan | .25 | .25 |
| Propylene Glycol USP (PG[USP]) | 3 | 3 |
| Hydroxyethyl cellulose (Natrosol 250 | .7 | -- |
| Guar gum (n-Hance HP40) | -- | .8 |
| Quaternary ammonium salt (Bardac 2250) | 2 | 2 |
| Cocamine oxide (Mackamine CO) | 13.3 | 13.3 |
| Cocamide (Cocamide DEA) | 2.25 | -- |
| Fragrance | .1 | -- |
| Pearlizing Agent | .5 | .5 |
| Lactic Acid pH = 6.0 | .3 | .3 |
| Water | balance to provide 100 percent by weight | |

These formulations were then evaluated and compared to several commercially available triclosan antimicrobial skin cleansers. More particularly, two professional antimicrobial lotion soaps were obtained from local medical distributors and tested against the two antimicrobial soap formulations of the present invention described hereinabove in Table V. While the exact compositional formulations of the commercially available antimicrobial lotion soaps are unknown or proprietary to the manufacturer, they are known to include triclosan as their active antimicrobial ingredient. However, as was the case with earlier PCMX formulations, upon examination of the ingredients for the commercial antimicrobial products, it was observed that they also contained at least one of ingredient believed to inhibit the antimicrobial activity of triclosan. In contrast, the triclosan formulations based upon the present invention employ only primary surfactants, which may include quaternary ammonium salts when used with triclosan, which are believed not to exhibit any inhibition to triclosan.

Each of the commercial antimicrobial soaps (Compositions 11 and 12) and the two triclosan Compositions, Nos. 9 and 10, were tested against *Staphylococcus aureus* (ATCC 33591 or ATCC 6538), *Escherichia coli* (ATCC 11229), *Pseudomonas aeruginosa* (ATCC 15442), and, in one instance, *Salmonella typhimurium* (ATCC 14028). Table VI reports the antibacterial efficacy of reduction of colony forming units (cfu's) of the challenged bacteria in terms of log reduction for a 30 second exposure screen performed against these bacteria.

**TABLE VI**

| **ANTIBACTERIAL EFFICACY DATA OF TWO COMMERCIAL AND TWO TRICLOSAN ANTIMICROBIAL SKIN CLEANSERS** | | | | |
|---|---|---|---|---|
| **Composition** | **LOG REDUCTION**^{**a**} | | | |
| | ***Staphylococcus aureus*** **[ATCC #33591 (A) #6538 (B)]** | ***Escherichia coli*** **[ATCC #11229]** | ***Pseudomonas aeruginosa*** **[ATCC #15442]** | ***Salmonella typhimurium*** **[ATCC #14028]** |
| **9** | >5.5091 (B) | >5.3502 | >5.7709 | >5.7709 |
| **10** | >5.3522 (A) | >5.1658 | >5.1004 | -- |
| **11** | 0.0770 (B) | -- | -- | -- |
| **12** | 0.2915 (A) | 0.7559 | >5.1004 | -- |

| | | | | |
|---|---|---|---|---|
| a = average of 9 replicates | | | | |

As can be seen in Table VI, the antibacterial efficacy of the compositions varies widely for each product. These results appear to indicate that the antimicrobial efficacies of antimicrobial skin cleansers are highly dependent upon the formula ingredients. Moreover, it appears that the inhibition of triclosan by formula ingredients can negate the effectiveness of certain compositions containing triclosan.

Of the commercial products, Composition 12 exhibited the highest antibacterial efficacy, showing greater than 5 log reductions for one of three challenge bacteria. However, the Compositions 9 and 10 of the present invention containing 0.25 percent by weight triclosan, exhibited greater than 5 log reductions for all three or all four challenge bacteria.

Thus, it is believed evident that the *in vitro* antibacterial efficacies of antimicrobial skin cleansers containing triclosan are also highly dependent upon the formula ingredients. Moreover, inhibition of triclosan by formula ingredients can negate the effectiveness of triclosan.

Thus it should be evident that the compositions of the present invention are highly effective in killing bacteria and other microorganisms while maintaining low skin irritation. The invention is particularly suited for hospital and other health care givers, but is not necessarily limited thereto. The compositions of the present invention can also be used with other surfactant or other ingredients including but not limited to fragrances, chelating and sequestering agents, perfumes, coloring agents, thickeners, antioxidants, emollients, and the like, which do not materially effect the cleansing and disinfecting nature of the composition.

Based upon the foregoing disclosure, it should now be apparent that the use of the composition described herein will carry out the objects set forth hereinabove. It is, therefore, to be understood that any variations evident fall within the scope of the claimed invention and thus, the selection of specific component elements can be determined without departing from the spirit of the invention herein disclosed and described. In particular, primary surfactants according to the present invention are not necessarily limited to those presented in the Examples. Any primary surfactant which, when added to a composition according to the present invention, provides a composition which can be shown to exhibit a log reduction value of at least 3 and preferably at least 5, against *Staphylococcus aureus* is believed suitable for the present invention. Moreover, as noted hereinabove, other viscosifiers and thickeners can be substituted for the hydroxyethyl cellulose. Thus, the scope of the invention shall include all modifications and variations that may fall within the scope of the attached claims.

## Claims

1. An antimicrobial cleansing composition comprising:
a substituted phenol; and
at least one primary surfactant selected from the group consisting of amine oxides, phospholipids, partially neutralized carboxylic acids and diacids, betaines, ethoxylated methyglucosides, and mixtures thereof;
said composition being devoid of anionic and nonionic surfactants detrimental to the activity of said substituted phenol.

2. An antimicrobial cleansing composition, as set forth in claim 1, wherein said substituted phenol is selected from the group consisting of alkyl substituted, halogen substituted, phenol substituted, alkyl halo substituted, halo phenol substituted, benzyl alkyl substituted, phenylethyl alkyl substituted and benzyl halo substituted phenols, and alkyl substituted, halogen substituted and alkyl halo substituted bisphenols, bis(hydroxyphenyl) alkanes, amino substituted phenols, resorcinol derivatives, trihydric phenol derivatives, naphthol derivatives and carboxylic acid substituted phenols (*e.g.*, salicylic acid and esters).

3. An antimicrobial cleansing composition, as set forth in claim 2, wherein said substituted phenol is selected from the group consisting of *p*-chloro-*m*-xylenol and triclosan.

4. An antimicrobial cleansing composition, as set forth in claim 1, wherein said at least one primary surfactant is an amine oxide having an alkyl chain length of from about C₈ to about C₁₆.

5. An antimicrobial cleansing composition, as set forth in claim 4, wherein said amine oxide is selected from the group consisting of alkylamine oxide and amidopropyl amine oxide.

6. An antimicrobial cleansing composition, as set forth in claim 1, wherein said at least one primary surfactant is a betaine selected from the group consisting of alkylammonio carboxylates having from 8 to 18 carbons atoms.

7. An antimicrobial cleansing composition, as set forth in claim 6, wherein said alkyl ammonio carboxylate having from 8 to 18 carbons atoms is cocobetaine.

8. An antimicrobial cleansing composition, as set forth in claim 1, wherein said at least one primary surfactant is a phospholipid selected from the group consisting of alkyl phosphatidyl PG-dimonium chloride.

9. An antimicrobial cleansing composition, as set forth in claim 1, further comprising a polymeric viscosifier.

10. An antimicrobial cleansing composition, as set forth in claim 9, wherein said polymeric viscosifier includes hydroxyethyl cellulose.

11. An antimicrobial cleansing composition, as set forth in claim 1, further comprising an acid to adjust the pH balance of the composition.

12. An antimicrobial cleansing composition, as set forth in claim 11, wherein said acid is selected from the group consisting of lactic acid, citric acid, acetic acid, glycolic acid, and gluconic acid.

13. An antimicrobial cleansing composition, as set forth in claim 1, having a pH between about 4 and 8.

14. An antimicrobial cleansing composition, as set forth in claim 1, further comprising water.

15. An antimicrobial cleansing composition, as set forth in claim 1, wherein said substituted phenol is present in an amount ranging between about 0.01 percent by weight and about 4 percent by weight of the total composition.

16. An antimicrobial cleansing composition, as set forth in claim 1, wherein said at least primary surfactant is present in an amount ranging from about 1 percent by weight to about 10 percent by weight of the total composition.

17. An antimicrobial cleansing composition, as set forth in claim 1, further comprising at least one additive selected from the group consisting of fragrances, chelating and sequestering agents, perfumes, coloring agents, opacifying agents, pearlizing agents, vitamins, antioxidants, emollients and skin care additives.

18. An antimicrobial cleansing composition, as set forth in claim 1, further comprising a water softening agent selected from the group consisting of chelating and sequestering agents.

19. An antimicrobial cleansing composition, as set forth in claim 18, wherein said water softening agent is ethylenediaminetetraacetic acid.
